# EUROPEAN PATENT APPLICATION

(11) **EP 4 574 133 A1**
(43) Date of publication of application: **25.06.2025**
(21) Application number: 23921576.7
(22) Date of filing: 05.12.2023
(51) Int. Cl.: A61K 9/00, A61K 47/36, A61K 47/38, A61K 47/26, A61K 47/18, A61K 38/26, A61P 3/04

(54) **COMPOSITION FOR LIRAGLUTIDE-CONTAINING MICRONEEDLE WITH ENHANCED STABILITY AND USE THEREOF**

(30) Priority: 25.10.2023 KR 20230144088
(71) Applicant: Small'Lab Co., Ltd., Daejeon 34-027 (KR)
(72) Inventor: KIM, Byeong Su, Daejeon 34011 (KR); JOO, So Kyoung, Seongnam-si, Gyeonggi-do 13490 (KR); CHOI, Hyuck, Namyangju-si, Gyeonggi-do 12221 (KR); PARK, Hyeonjun, Suwon-si, Gyeonggi-do 16460 (KR); LEE, Jeong Gyu, Daejeon 34049 (KR); PARK, Chun Woong, Sejong 30063 (KR)
(74) Representative: Ter Meer Steinmeister & Partner
(86) International application number: PCT/KR2023/019836
(87) International publication number: WO 2025/089491

(57) **Abstract**

The present invention provides a composition for manufacturing liraglutide-containing microneedles that contains liraglutide as an active ingredient, without denaturation of the active ingredient during manufacture, and maintains stability even after manufacture, and liraglutide-containing microneedles with enhanced stability manufactured using the composition.

The composition for manufacturing liraglutide-containing microneedles according to the present invention uses a specific combination of a biodegradable polymer, a stabilizer and an antioxidant to prevent the denaturation of the active ingredient liraglutide during the manufacture of microneedles and to ensure that the stability of liraglutide is maintained after the manufacture of microneedles.

## Description

### Technical Field

The present invention relates to a composition for liraglutide-containing microneedles with enhanced stability and use thereof, more specifically to a composition for manufacturing liraglutide-containing microneedles that contains liraglutide as an active ingredient, without denaturation of the active ingredient during manufacture, and maintains stability even after manufacture, and liraglutide-containing microneedles with enhanced stability manufactured using this composition.

### Background Art

Liraglutide is a human glucagon-like peptide(GLP)-1 analogue that binds to and activates the GLP-1 receptor to increase glucose-dependent insulin secretion and inhibit inappropriate glucagon secretion. It is used for the treatment of type 2 diabetes and obesity. Liraglutide is marketed under the brand names Victoza for type 2 diabetes and Saxenda for obesity.

Currently, liraglutide is only used as a subcutaneous injection, which has the inconvenience of having to disinfect the injection site and administer the injection at a set time every day, as well as concerns about secondary infection. Moreover, repeated injections can cause pain, bleeding, rash, and swelling at the injection site. In the case of self-injectable medications, patients have to administer the injection themselves, which can lead to fear and aversion, resulting in decreased medication adherence and consequently reduced therapeutic efficacy.

To address these problems, some attempts have been made to formulate liraglutide as microneedles. However, only a method for manufacturing liraglutide-containing microneedles that do not bounce off the skin surface, do not break or bend, and can be mass-produced by shortening the drug manufacturing time (Korean Patent Application Publication No. 10-2022-0151995) has been disclosed.

Meanwhile, liraglutide has poor stability and is susceptible to denaturation by various factors during the manufacturing process of microneedles, and its activity can be reduced by denaturation during storage after manufacturing.

Therefore, there is a need for the development of liraglutide-containing microneedles that prevent denaturation of liraglutide during manufacturing to retain activity and prevent denaturation of liraglutide during storage after manufacturing to enhance stability.

### Disclosure of the Invention

### Technical Problems to be Solved

The present inventors continued their research to meet the needs of the prior art and found that liraglutide-containing microneedles manufactured using a specific combination of a biodegradable polymer, a stabilizer, and an antioxidant surprisingly prevent denaturation of liraglutide during manufacturing to retain activity and prevent denaturation of liraglutide during storage after manufacturing to enhance stability, thereby completing the present invention.

Accordingly, it is an object of the present invention to provide a composition for manufacturing liraglutide-containing microneedles with enhanced stability.

Another object of the present invention is to provide liraglutide-containing microneedles with enhanced stability manufactured using said composition.

Another object of the present invention is to provide a microneedles patch for transdermal delivery of liraglutide comprising said microneedles.

### Technical Solutions

To achieve the objects of the present invention, a composition for manufacturing liraglutide-containing microneedles with enhanced stability is provided.

A composition for manufacturing liraglutide-containing microneedles with enhanced stability according to the present invention comprises liraglutide, a biodegradable polymer, a stabilizer, an antioxidant, and a solvent.

Liraglutide has the structure of Formula 1 and is a human glucagon like peptide (GLP)-1 analog that binds to and activates the GLP-1 receptor, increasing glucose-dependent insulin secretion and inhibiting inappropriate glucagon secretion, and is used for the treatment of type 2 diabetes and obesity:

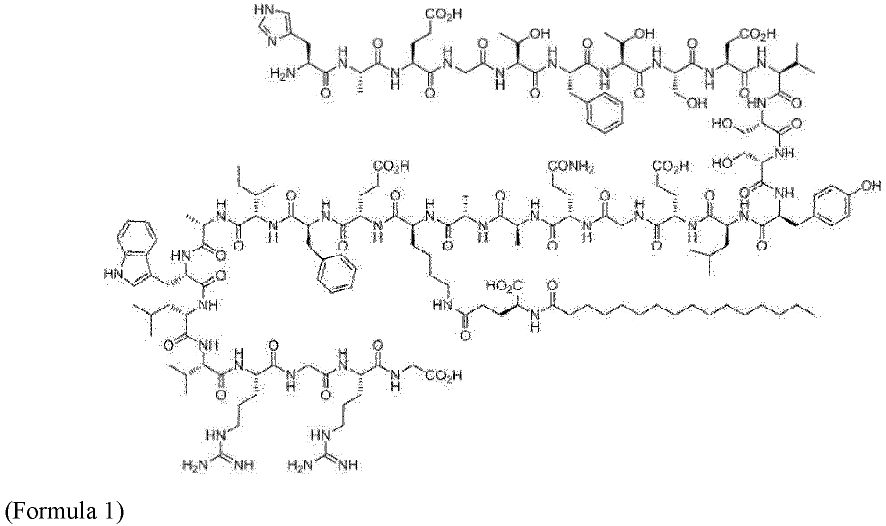

In the present invention, a biodegradable polymer is one that can be naturally biodegraded in the body, and may be at least one selected from the group consisting of hyaluronic acid or a salt thereof and carboxymethyl cellulose (CMC), most preferably hyaluronic acid or a salt thereof. Compared to other types of biodegradable polymers, compositions for manufacturing microneedles containing these biodegradable polymers can prevent denaturation of liraglutide during microneedle manufacturing and storage, thereby increasing stability.

In a composition of the present invention, the biodegradable polymer may be comprised from in an amount of 10 wt% to 20 wt% based on the total of the composition (100 wt%).

In the present invention, a stabilizer serves to aid in stability during manufacturing of the microneedles and may be at least one selected from the group consisting of xylitol and trehalose. Compared to other types of stabilizers, compositions for manufacturing microneedles containing these stabilizers enable stable manufacturing of microneedles while preventing denaturation of liraglutide during microneedle manufacturing and storage to enhance stability.

In the present invention, the stabilizer may be comprised from in an amount of 3 wt% to 10 wt% based on the total of the composition.

In the present invention, an antioxidant may be at least one selected from the group consisting of dipotassium glycyrrhizinate (DPG) and EDTA. Compared to other types of antioxidants, compositions for manufacturing microneedles containing these antioxidants can prevent denaturation of liraglutide during microneedle manufacturing and storage to enhance stability.

In a composition of the present invention, the antioxidant may be comprised relative to the weight of liraglutide, i.e., in a weight ratio of liraglutide : antioxidant of 1 : 0.3 to 5.

In a composition of the present invention, liraglutide may be comprised from in an amount of 0.1 wt% to 2.0 wt% based on the total of the composition.

In the compositions of the present invention, the solvent is water, preferably deionized water, or potassium phosphate buffer (PPB).

A composition of the present invention may further comprise a pH adjusting agent as desired. The pH adjusting agent can be any of those commonly used in the manufacture of microneedles, such as NaOH. A composition according to the present invention has a pH of 7.5 to 8.7, preferably 7.8 to 8.4.

A composition of the present invention may further comprise plasticizers, surfactants, preservatives, and the like that may be conventionally used in the manufacture of microneedles, as needed.

In accordance with another object of the present invention, the present invention provides liraglutide-containing microneedles with enhanced stability prepared with a composition according to the present invention.

Microneedles of the present invention may comprise needle parts protruding in one direction and a mattress layer supporting the needle parts. The needle parts have a shape suitable for penetrating the skin. The shape of the needle parts of the microneedles according to the present invention may be conical, pyramidal, spherical, truncated, wedge-shaped, blade-shaped, etc., which all have a shape capable of penetrating the skin. The needle parts have a length of 500 to 1000 µm, preferably 750 µm. The mattress layer has a thickness of 0.1 to 1 mm, preferably 0.1 to 0.3 mm. If needed, the needle parts of the microneedles of the present invention may be manufactured to separate from the mattress layer when inserted into the skin. A composition according to the present invention may be used to manufacture the needle parts and mattress layer of the microneedles, and if needed, the mattress layer may be manufactured from a different material. Therefore, in the microneedles of the present invention, liraglutide may be evenly distributed in the needle parts and mattress layer, or liraglutide may be distributed only in the needle parts.

Microneedles of the present invention are dissolvable microneedles that degrade in vivo while releasing liraglutide.

Microneedles of the present invention can be manufactured using a mold.

In accordance with another object of the present invention, there is provided a microneedle patch for transdermal delivery of liraglutide comprising microneedles according to the present invention.

A microneedle patch of the present invention has an adhesive layer laminated to one side of the mattress layer, which allows the microneedle patch to be applied to the skin. The microneedle patch may also comprise a protective film over the adhesive layer.

A microneedle patch for transdermal delivery of liraglutide of the present invention can be used for the treatment and amelioration of obesity and for the treatment and amelioration of type 2 diabetes.

### Advantageous Effects

A composition for manufacturing microneedles according to the present invention uses a specific combination of a biodegradable polymer, a stabilizer, and an antioxidant to prevent the denaturation of the active ingredient liraglutide during manufacturing microneedles and to maintain the stability of liraglutide after the manufacture of microneedles.

Microneedles according to the present invention are liraglutide-containing microneedles with enhanced stability that prevent denaturation of liraglutide during manufacturing to retain activity and prevent denaturation of liraglutide during storage after manufacturing.

A microneedle patch for transdermal delivery of liraglutide according to the present invention has enhanced stability of the active ingredient liraglutide and is useful for the treatment and amelioration of obesity and for the treatment and amelioration of type 2 diabetes.

### Brief Description of Drawings

FIG. 1 is a graph showing the results of a stability analysis for each biodegradable polymer used.
FIG. 2 is a graph showing the results of a stability analysis for each stabilizer used.
FIG. 3 is a photograph of microneedles according to the present invention.
FIG. 4 is a graph showing the results of a stability analysis of microneedles according to the present invention.

### Detailed Description of the Invention

Hereinafter, the present invention will described in more detail through the following examples. However, the following examples are provided only for the purpose of illustrating the present invention, and thus the scope of the present invention is not limited thereto.

### Preparation Example 1: Preparation of compositions for manufacturing microneedles by using various biodegradable polymers

To select a biodegradable polymer suitable for manufacturing of liraglutide-containing microneedles with enhanced stability, compositions for manufacturing microneedles were prepared with each biodegradable polymer shown in Table 1 and prepared into thin films to test stability.

**Table 1**

| Content (mg) | Composition 1 | Composition 2 | Composition 3 | Composition 4 | Composition 5 |
|---|---|---|---|---|---|
| Liraglutide (Lira) | 10.0 | 10.0 | 10.0 | 10.0 | 10.0 |
| Sodium Alginate (Alginate) | - | 30.0 | - | - | - |
| Sodium Hyaluronate (HA) | - | - | 30.0 | - | - |
| Carboxy methyl cellulose(CMC) | - | - | - | 30.0 | - |
| Polyvinyl pyrrolidone (PVP) | - | - | - | - | 30.0 |

Specifically, 1 ml of deionized water (DW), liraglutide and biodegradable polymer weighed as shown in Table 1 were put into a cup tube and dissolved using a vortex mixer for 5 minutes, and dried at 25°C for 24 hours using a desiccator. Then, the solvent was completely evaporated using an evaporator under nitrogen to prepare a film (50~100 µm thick) that can mimic microneedles.

The prepared films were analyzed for stability immediately after preparation and after storage for 1 week under accelerated conditions of 25°C, 60% in a temperature and humidity controlled stability chamber.

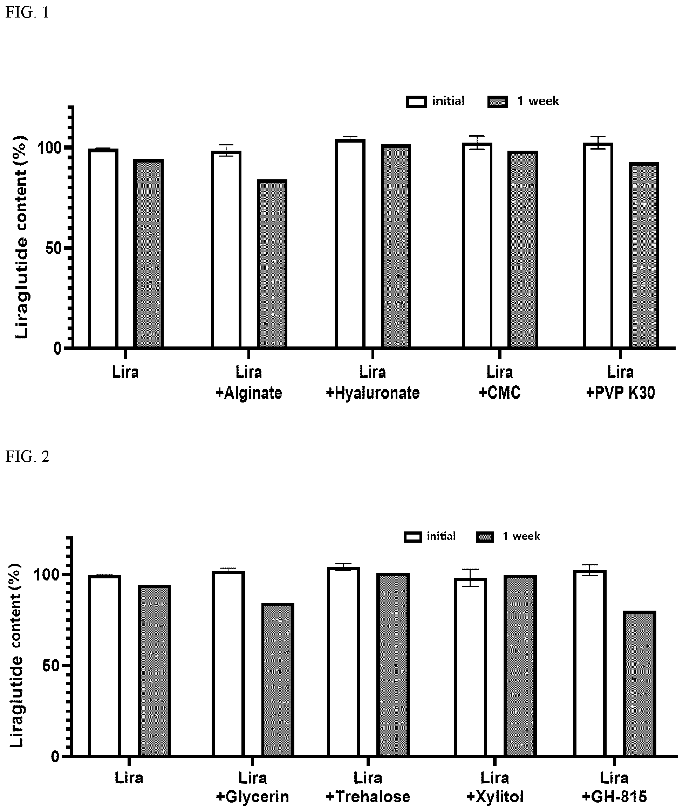

Specifically, for stability analysis, each film sample was added to 5 ml of dilution solvent (1: 1 mixture of phosphate buffer solution pH 3.5 : 78% acetonitrile) and shaken to mix, then quantitatively analyzed for liraglutide using the HPLC method under the conditions shown in Table 2 below, and the results are shown in Table 3 and Fig. 1.

**Table 2**

| | |
|---|---|
| Devices | Thermo U3000 |
| Columns | C8, 4.6 * 100 mm, 3.5 µm |
| Wavelength | 215 nm |
| Column Temperature | 45 |
| Flow rate | 1.0 ml/min |
| Injection volume | 10 µl |
| Run time | 40 min |
| Mobile Phase | A: Phosphate buffer solution (pH 3.5) |
| | B: 78% Acetonitrile |
| Gradient | 0-40 min: 56-64% B |

**Table 3**

| Content (%) | Composition 1 (Lira) | Composition 2 (Lira+Alginate) | Composition 3 (Lira+HA) | Composition 4 (Lira+CMC) | Composition 5 (Lira+PVP) |
|---|---|---|---|---|---|
| Immediately after preparation | 99.5±0.3 | 98.6±2.8 | 104.3±1.2 | 102.4±3.3 | 98.2±4.7 |
| After 1 week of storage | 94.2±3.7 | 84.2±9.6 | 101.5±3.8 | 98.4±2.4 | 92.7±3.8 |

As shown in Table 3 and FIG. 1, it can be confirmed that the use of hyaluronic acid and CMC among the biodegradable polymers enhanced stability by preventing the denaturation of liraglutide immediately after film preparation and after 1 week of storage. When alginate or PVP were used as biodegradable polymers, some denaturation of liraglutide was seen immediately after preparation and after 1 week of storage. Therefore, it can be confirmed that hyaluronic acid and CMC are suitable as biodegradable polymers for liraglutide-containing microneedles, with hyaluronic acid being the most suitable.

### Preparation Example 2: Preparation of compositions for manufacturing microneedles by using various stabilizer

To select a stabilizer suitable for manufacturing liraglutide-containing microneedles with enhanced stability, compositions for manufacturing microneedles were prepared with each stabilizer shown in Table 4 and prepared into thin films in the same manner as in Preparation Example 1, and stability analysis was performed in the same manner as in Preparation Example 1.

**Table 4**

| Content (mg) | Composition 1 (Lira) | Composition 6 Lira+Glycerin | Composition 7 Lira+Trehalose | Composition 8 Lira+xylitol | Composition 9 Lira+GH815 |
|---|---|---|---|---|---|
| Liraglutide (Lira) | 10.0 | 10.0 | 10.0 | 10.0 | 10.0 |
| Glycerin | - | 30.0 | - | - | - |
| Trehalose | - | - | 30.0 | - | - |
| Xylitol | - | - | - | 30.0 | - |
| GH-815 (Propanediol , Caprylyl Glycol, Ethylhexylglycerin) | - | - | - | - | 10.0 |

The results are shown in Table 5 and FIG. 2.

**Table 5**

| Content (%) | Composition 1 (Lira) | Composition 6 Lira+Glycerin | Composition 7 Lira+Trehalose | Composition 8 Lira+xylitol | Composition 9 Lira+GH815 |
|---|---|---|---|---|---|
| Immediately after preparation | 99.5±0.3 | 102.0±1.5 | 104.2±1.9 | 98.2±4.7 | 102.4±3.0 |
| After 1 week of storage | 94.2±3.7 | 84.6±5.3 | 100.9±0.7 | 99.7±3.8 | 80.3±5.8 |

As shown in Table 5 and FIG. 2, it can be confirmed that the use of trehalose and xylitol as stabilizers enhanced stability by preventing liraglutide denaturation immediately after film preparation and after 1 week of storage. When glycerin or GH-815 (a mixture of propanediol, caprylyl glycol, and ethylhexylglycerin) was used as a stabilizer, denaturation of liraglutide occurred after 1 week of storage. Therefore, it can be confirmed that trehalose and xylitol are suitable as stabilizers for liraglutide-containing microneedles.

### Preparation Example 3: Preparation of compositions for manufacturing microneedles by using various antioxidants

To select an antioxidant suitable for manufacturing liraglutide-containing microneedles with enhanced stability, compositions for manufacturing microneedles were prepared using hyaluronic acid as the biodegradable polymer along with each antioxidant shown in Table 6 and prepared into thin films in the same manner as in Preparation Example 1, and stability analysis was performed in the same manner as in Preparation Example 1.

**Table 6**

| Content (mg) | Composition 10 Lira + HA | Composition 11 Lira + HA + BHA | Composition 12 Lira + HA + BHT | Composition 13 Lira + HA + EDTA | Composition 14 Lira + HA + DPG |
|---|---|---|---|---|---|
| Liraglutide (Lira) | 3.00 | 3.00 | 3.00 | 3.00 | 3.00 |
| HA | 2.82 | 2.82 | 2.82 | 2.82 | 2.82 |
| BHA (Butylated hydroxyanisole) | - | 0.3 | - | - | - |
| BHT (Butylated hydroxytoluene) | - | - | 0.3 | - | - |
| EDTA (Disodium Dihydrogen Ethylene-diaminetetra-acetate Dihydrate) | - | - | - | 0.3 | - |
| DPG (Dipotassium glycyrrhizinate) | - | - | - | - | 0.3 |

The results are shown in Table 7.

**Table 7**

| Content (%) | Composition 10 Lira + HA | Composition 11 Lira + HA + BHA | Composition 12 Lira + HA + BHT | Composition 13 Lira + HA + EDTA | Composition 14 Lira + HA + DPG |
|---|---|---|---|---|---|
| Immediately after preparation | 86.8±2.4 | 79.8±7.3 | 76.4±5.3 | 89.3±1.2 | 88.3±2.1 |
| After 1 week of storage | 82.1±2.4 | 69.8±2.1 | 54.2±1.3 | 90.5±3.4 | 87.1±0.8 |

As shown in Table 7, it can be confirmed that the use of EDTA and DPG (Dipotassium glycyrrhizinate) among the antioxidants enhanced stability by preventing liraglutide denaturation immediately after film preparation and after 1 week of storage. When BHA (butylated hydroxyanisole) or BHT (butylated hydroxytoluene) were used as antioxidants, some denaturation of liraglutide was observed immediately after preparation and after 1 week of storage. Therefore, it can be confirmed that EDTA and DPG are suitable as antioxidants for liraglutide-containing microneedles.

### Preparation Example 4: Preparation of liraglutide-containing microneedles

### <Preparation of compositions for manufacturing microneedles>

Compositions for manufacturing microneedles were prepared with the compositions shown in Table 8:

**Table 8**

| Content (mg) | Example 1 | Example 2 | Example 3 | Example 4 |
|---|---|---|---|---|
| Liraglutide | 0.26 | 0.26 | 0.26 | 0.26 |
| HA | 39.29 | 39.29 | 39.29 | 39.29 |
| Xylitol | 13.10 | - | 13.10 | - |
| Trehalose | - | 13.08 | - | 13.10 |
| DPG | 0.26 | 0.26 | 0.26 | - |
| EDTA | - | - | - | 0.26 |
| 0.1 M NaOH aqueous solution | 41.65 | 41.65 | 41.065 | 41.65 |
| DW | The remaining | The remaining | - | - |
| 0.005M PPB | - | - | The remaining | The remaining |
| Total (mg) | 200 | 200 | 200 | 200 |

Specifically, for the compositions of Example 1 through Example 4, DPG or EDTA was put into a conical tube, then a solution prepared by mixing deionized water or potassium phosphate buffer (PPB) and 0.1M NaOH solution for 10 minutes by vortexing was added to the tube and vortexed for 10 minutes. Then, xylitol or trehalose was added and vortexed for 5 minutes, liraglutide was added and vortexed for 5 minutes, and sodium hyaluronate was added as hyaluronic acid (HA) and vortexed for 30 minutes to complete the compositions.

The prepared compositions had a pH of about 7.8 to 8.4.

### <Preparation of microneedles>

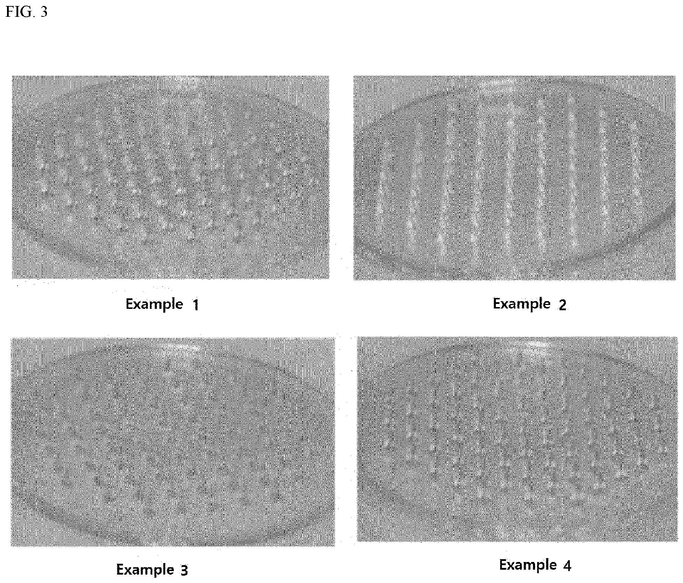

Each prepared composition was loaded into a silicon negative mold engraved with microneedle shapes. The loaded mold was placed in a desiccator, depressurized to -0.04 MPa and maintained for 5 minutes, then ventilated and bubbles generated were removed using an air gun. Then, the mold was placed in a hot air dryer and dried at 30°C for 2 hours, and the completed microneedles were separated from the mold using tweezers to obtain microneedles. Photographs of the prepared microneedles were taken and shown in Fig. 3.

As needed, the prepared microneedles can be made more usable in the form of a patch with a colloidal band on the back.

As shown in FIG. 3, it can be seen that microneedles are well formed when using the compositions of Examples 1-4.

The microneedles prepared with the compositions of Examples 1-4 also had sufficient strength to penetrate the skin.

### Test Example 1: Analysis of drug stability in microneedles

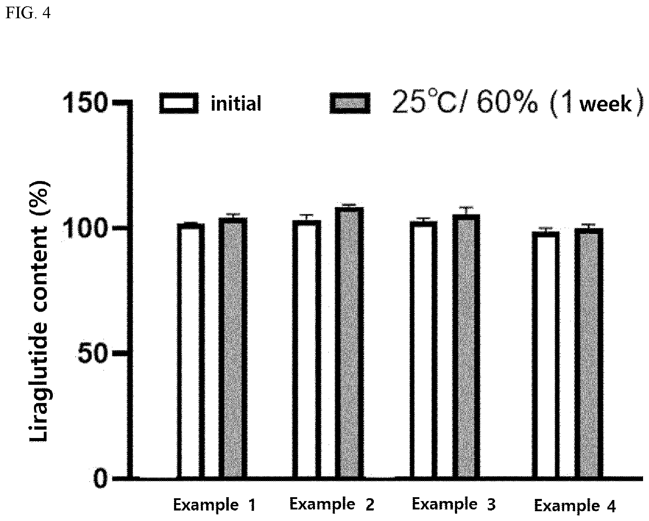

Stability analysis was performed in the same manner as in Preparation Example 1 for each of the microneedles of Examples 1 through 4 prepared in Preparation Example 4, and the results are shown in Table 9 and Fig. 4.

**Table 9**

| Content (%) | Example 1 | Example 2 | Example 3 | Example 4 |
|---|---|---|---|---|
| Immediately after preparation | 101.86±0.39 | 103.41±2.03 | 102.80±1.26 | 99.55±1.57 |
| After 1 week of storage | 104.36±1.38 | 108.54±0.78 | 105.64±2.65 | 99.93±1.68 |

As shown in Table 9 and FIG. 4, it can be seen that the microneedles of Example 1 to Example 3, which were prepared with compositions containing hyaluronic acid as the biodegradable polymer, xylitol or trehalose as the stabilizer, and DPG as the antioxidant, exhibited excellent stability with prevented denaturation of liraglutide immediately after preparation, and maintain excellent stability even after 1 week of storage. It can also be seen that the microneedles of Example 4, prepared with a composition containing EDTA as the antioxidant, maintain excellent stability with prevented denaturation of liraglutide after 1 week of storage.

Therefore it can be confirmed that the stability of liraglutide-containing microneedles is enhanced only when using the specific combination of biodegradable polymer, stabilizer, and antioxidant according to the present invention.

## Claims

1. A composition for manufacturing liraglutide-containing microneedles with enhanced stability, wherein the composition comprises liraglutide, a biodegradable polymer, a stabilizer, an antioxidant, and a solvent,
the biodegradable polymer is at least one selected from the group consisting of hyaluronic acid or a salt thereof and carboxymethylcellulose (CMC),
the stabilizer is at least one selected from the group consisting of xylitol and trehalose, and
the antioxidant is at least one selected from the group consisting of DPG (Dipotassium glycyrrhizinate) and EDTA.

2. The composition for manufacturing liraglutide-containing microneedles with enhanced stability according to claim 1, wherein the biodegradable polymer is hyaluronic acid or a salt thereof.

3. The composition for manufacturing liraglutide-containing microneedles with enhanced stability according to claim 1, wherein the stabilizer is xylitol.

4. The composition for manufacturing liraglutide-containing microneedles with enhanced stability according to claim 1, wherein the antioxidant is DPG.

5. The composition for manufacturing liraglutide-containing microneedles with enhanced stability according to claim 1, wherein the biodegradable polymer is hyaluronic acid or a salt thereof, the stabilizer is xylitol, and the antioxidant is DPG.

6. The composition for manufacturing liraglutide-containing microneedles with enhanced stability according to claim 1, wherein the biodegradable polymer is hyaluronic acid or a salt thereof, the stabilizer is trehalose, and the antioxidant is DPG.

7. The composition for manufacturing liraglutide-containing microneedles with enhanced stability according to claim 1, wherein the biodegradable polymer is comprised in an amount of 10 wt% to 20 wt% based on the total weight of the composition.

8. The composition for manufacturing liraglutide-containing microneedles with enhanced stability according to claim 1, wherein the stabilizer is comprised in an amount of 3 wt% to 10 wt% based on the total weight of the composition.

9. The composition for manufacturing liraglutide-containing microneedles with enhanced stability according to claim 1, wherein the antioxidant is comprised such that the weight ratio of liraglutide to antioxidant is 1 : 0.3 to 5.

10. Liraglutide-containing microneedles with enhanced stability manufactured using the composition according to claim 1, wherein the microneedles comprise needle parts protruding in one direction and a mattress layer supporting the needle parts.

11. The liraglutide-containing microneedles with enhanced stability according to claim 10, wherein the needle parts can separate from the mattress layer when inserted into the skin.

12. The liraglutide-containing microneedles with enhanced stability according to claim 10, wherein the microneedles are manufactured using a mold.

13. A microneedle patch for transdermal delivery of liraglutide, comprising the microneedles according to claim 10.

14. The microneedle patch for transdermal delivery of liraglutide according to claim 13, wherein the microneedle patch is for the treatment and amelioration of obesity.

15. The microneedle patch for transdermal delivery of liraglutide according to claim 13, wherein the microneedle patch is for the treatment and amelioration of type 2 diabetes.
